# EUROPÄISCHE PATENTSCHRIFT

(11) **EP 1 846 053 B1**
(45) Veröffentlichungstag und Bekanntmachung des Hinweises auf die Patenterteilung: **05.12.2012**
(21) Anmeldenummer: 06706604.3
(22) Anmeldetag: 03.02.2006
(51) Int. Cl.: A61L 17/12, A61L 17/14, D06M 11/05

(54) **RESORBIERBARES CHIRURGISCHES NAHTMATERIAL**
RESORBABLE SURGICAL SUTURE MATERIAL
MATERIAU DE SUTURE CHIRURGICALE RESORBABLE

(30) Priorität: 04.02.2005 DE 102005006718
(43) Veröffentlichungstag der Anmeldung: 24.10.2007
(73) Patentinhaber: Aesculap AG, 78532 Tuttlingen (DE)
(72) Erfinder: ODERMATT, Erich, K., CH-8200 Schaffhausen (CH); EGLOFF, Jürgen, 78532 Tuttlingen (DE); BARGON, Rainer, 78532 Tuttlingen (DE); MÜLLER, Erhard, 70565 Stuttgart (DE)
(74) Vertreter: Patentanwälte Ruff, Wilhelm, Beier, Dauster & Partner
(86) Internationale Anmeldenummer: PCT/EP2006/000935
(87) Internationale Veröffentlichungsnummer: WO 2006/082060

(56) Entgegenhaltungen:
- EP-A- 0 050 215
- EP-A- 0 999 227
- WO-A-2004/050127
- DE-C1- 19 702 708
- US-A- 3 225 766
- US-A- 4 135 622
- US-A1- 2004 220 614
- SHUTTLEWORTH G N ET AL: "Material properties of ophthalmic sutures after sterilization and disinfection." JOURNAL OF CATARACT AND REFRACTIVE SURGERY. SEP 1999, Bd. 25, Nr. 9, September 1999 (1999-09), Seiten 1270-1274, XP002391594 ISSN: 0886-3350
- anonymous: "Sterilization Cycles", , 14 March 2012 (2012-03-14), pages 1-3, Retrieved from the Internet: URL:http://consteril.com/index.php?pg=41 [retrieved on 2012-03-14]
- anonymous: "Autoclave", , 9 March 2012 (2012-03-09), pages 1-4, Retrieved from the Internet: URL:http://en.wikipedia.org/wiki/Autoclave [retrieved on 2012-03-14]

## Beschreibung

Die Erfindung betrifft resorbierbares, chirurgisches Nahtmaterial auf Basis von Polyglykolsäure oder auf Basis eines Lactidcopolymers. Das Nahtmaterial weist "in vivo" einen beschleunigten Abbau auf. Weiterhin betrifft die Erfindung ein Verfahren zur Herstellung von beschleunigt resorbierbarem, chirurgischem Nahtmaterial.

Resorbierbare Nahtmaterialien werden nach dem Einbringen in den Körper im Laufe der Zeit resorbiert. Zu den "herkömmlichen" resorbierbaren Nahtmaterialien sind mittlerweile insbesondere Polymerisate und Copolymerisate aus α-Hydroxysäuren, insbesondere aus den Bausteinen Glykolsäure und Milchsäure, zu zählen.

Polylactide sind bekanntlich biologisch abbaubare Polyester auf Basis von Milchsäure. Sie liegen gewöhnlich in zwei optischen Isomeren vor, der Poly-L-Milchsäure und der Poly-D,L-Milchsäure. Während erstere teilkristallin, relativ hart und spröde ist, ist letztere eher amorph und biegsam. Allgemein läßt sich bereits durch definierte Copolymerisation dieser beiden Isomere das Abbauverhalten des resultierenden Polylactids voreinstellen.

Im Körper wird resorbierbares Nahtmaterial auf Basis der genannten Polymerisate und Copolymerisate gewöhnlich durch Hydrolyse, im Fall von Polylactiden auch enzymatisch abgebaut. Die entstehenden Abbauprodukte, Glykolsäure und Milchsäure, werden im Körper metabolisiert bzw. gegebenenfalls nicht metabolisiert ausgeschieden.

Als resorbierbares Nahtmaterial hat sich beispielsweise Safil® bewährt, ein von der Anmelderin vertriebenes Nahtmaterial. Safil® ist ein mittelfristig resorbierbares, synthetisches, geflochtenes und beschichtetes Nahtmaterial aus Polyglykolsäure (Polyglykolid) das beispielsweise violett eingefärbt und auch ungefärbt bekannt ist. Safil® wird durch Hydrolyse vorhersagbar und zuverlässig abgebaut und resorbiert.

Ein Beispiel für Nahtmaterial aus den Bausteinen Milchsäure und Glykolsäure ist das Copolymer aus Glykolid und Lactid im Verhältnis 9 : 1, das von der Firma Ethicon unter der Bezeichnung Vicryl® vertrieben wird.

Von der Firma Tyco wird resorbierbares, geflochtenes Nahtmaterial unter der Handelsbezeichnung Polysorb® vertrieben, auch bei diesem handelt es sich um ein Copolymer auf Basis von Glykolid und Lactid.

Resorbierbare Nahtmaterialien unter anderem auf Basis von Lactidcopolymeren sind in der EP 999227 A2 beschrieben.

In der Chirurgie werden für Indikationen, bei denen eine Naht schon nach kurzer Zeit ihren Zweck erfüllt hat, häufig Nahtmaterialien eingesetzt, die nach dem Einbringen in den Körper schneller resorbiert werden, als dies bei herkömmlichen resorbierbaren Nahtmaterialien der Fall ist.

Es sind verschiedene Vorgehensweisen bekannt, um die Resorptionsdauer von herkömmlichem resorbierbarem Nahtmaterial zu verkürzen. Die wichtigsten werden im folgenden kurz erläutert.

Nach einem weit verbreiteten Verfahren wird das Nahtmaterial während oder nach der Fertigung vorzugsweise mit Gammastrahlung bestrahlt. Die Strahlung erzeugt Defekte in der Polymerstruktur des Nahtmaterials und führt dadurch zu einem schnelleren Reißfestigkeitsabfall und einer kürzeren Resorptionsdauer des Nahtmaterials "in vivo". Eine solche Behandlung ist mit hohem anlagentechnischem Aufwand und aufwendigen Arbeitsschutzvorkehrungen verbunden. Auch ist bei einem Bestrahlungsverfahren die Anfangsknotenreißkraft (abhängig von der Fadenstärke) nicht zufriedenstellend. Zudem können bei Bestrahlen von eingefärbtem Nahtmaterial Probleme auftreten, so ist beispielsweise der violette Farbstoff D&C violett No.2 unter Bestrahlung mit Gammastrahlen nicht persistent.

In der DE 197 02 708 wird ein Verfahren zur Hydrolyse von Nahtmaterial in verschiedenen Puffersystemen beschrieben. Vorzugsweise wird das Verfahren im Rahmen des Produktionsprozesses im Anschluß an das Spinnen, Flechten, Verstrecken und einem Tempern, das bei Temperaturen zwischen 70° bis 120°C erfolgt, durchgeführt. Dabei wird resorbierbares chirurgisches Nahtmaterial in einem Inkubationsbad bei einer Temperatur im Bereich von 30°C bis 65°C über einen Zeitraum von mindestens 10 Stunden, bevorzugt mehr als 30 Stunden inkubiert. Die Wahl der Verfahrensparameter Temperatur, Zeit und PH-Wert beeinflußt die Abbaubarkeit des resultierenden Nahtmaterials sowie den Reißfestigkeitsabfall "in vitro" und "in vivo". Die genannten Parameter müssen daher penibel überwacht werden. Das Verfahren ist mit einer Dauer von bis zu mehreren Tagen zudem sehr zeitaufwendig.

In der WO 2004/050127 wird ebenfalls ein Verfahren beschrieben, bei dem bioabsorbierbares Material wie das oben bereits erwähnte Polysorb® einem hydrolytischen Teilabbau ausgesetzt wird. Dazu wird das Material in eine Klimakammer eingebracht, in der es über einen Zeitraum von vorzugsweise 5 bis 8 Tagen bei 20 % bis 70 % relativer Luftfeuchtigkeit (bevorzugt zwischen 45 % und 55 %) und einer maximalen Temperatur von 93 °C (200 °F) ruht. Als bevorzugter Temperaturbereich wird der Bereich zwischen 52 °C und 57 °C angegeben. Das Verfahren ist allgemein zur Behandlung chirurgischer Artikel, insbesondere auch zur Behandlung von Nahtmaterial, vorgesehen- Auch hier ist insbesondere der hohe Zeitaufwand, der zur Durchführung des Verfahrens erforderlich ist, als nachteilig anzusehen.

In der EP 0 050 215 A1 wird Polyglykolsäure, welche mit einem Füllmaterial wie beispielsweise Bariumsulfat compoundiert ist, einer hydrolytischen Behandlung unterworfen.

Gegenstand der US 3,225,766 ist ein Herstellungsverfahren für Prothesen und chirurgische Nahtmaterialien auf der Basis von bakteriell hergestellter Poly-β-Hydroxysäure, wobei die Prothesen und chirurgischen Nahtmaterialien mittels Autklavieren sterilisiert werden.

In dem Artikel "Material properties of ophthalmic sutures after sterilization and disinfection" von Shuttleworth et al. (Journal of Cataract and refractive surgery, Sep. 1999, Bd. 25, Nr. 9, Seiten 1270-1274) werden die Ergebnisse einer Studie über die Reißfestigkeit von verschiedenen ophthalmischen Nahtmaterialien in Abhängigkeit von verschiedenen Sterilisierungs- und Desinfektionsmethoden beschrieben.

Aus der US 4,135,622 ist eine Verpackungsform bekannt, welche ein steriles chirurgisches Implantat auf der Basis von Polyglykolsäure enthält.

Die US 2004/0220614 A1 betrifft verpackte Nahtmaterialien, wobei die Nahtmaterialien aus einem resorbierbaren Polymer, beispielsweise auf der Basis von Laktid- und/oder Glykolid, bestehen.

Es ist die Aufgabe der Erfindung, ein resorbierbares Nahtmaterial bereitzustellen, dessen Knotenreißkraft "in vivo", also nach dem Implantieren, schnell abfällt. Insbesondere ist es auch Aufgabe der Erfindung, ein Verfahren zur Herstellung von beschleunigt resorbierbarem, chirurgischem Nahtmaterial bereitzustellen, das die genannten Nachteile bereits bekannter Verfahren vermeidet. Das Verfahren soll insbesondere möglichst einfach, schnell und damit kostengünstiger als die bekannten Verfahren durchführbar sein.

Diese Aufgabe wird erfindungsgemäß gelöst durch resorbierbares, thermisch dampfbehandeltes, chirurgisches Nahtmaterial auf Basis von Polyglykolsäure oder Lactidcopotymer gemäß Anspruch 1 mit einem schnellen Abbau und einem schnellen Abfall der Knotenreißkraft unter "in vivo"-Bedingungen und einer im Verhältnis zum schnellen Abbau hohen Anfangsknotenreißkraft.

Es wurde gefunden, daß Nahtmaterialien aus Polymeren auf Basis von Glykolsäure oder Polymeren auf Basis von Milchsäure nach einer Dampfbehandlung die gewünschten Eigenschaften zeigen, wogegen Nahtmaterialien aus Polymeren und Copolymeren anderer Hydroxysäuren hier versagen.

Geeignete Lactidcopolymere sind insbesondere in der EP 999227 A2 beschrieben und weisen einen mehrheitlichen Anteil an Lactid auf. Bei der bekannten Polyglykolsäure handelt es sich um einen Polyester auf Basis von Glykolsäure. Erfindungsgemäßes Nahtmaterial auf Basis von Polyglykolsäure ist besonders bevorzugt. Weiterhin kann es sich bei dem erfindungsgemäßen Nahtmaterial auf Basis von Polyglykolsäure um ein Nahtmaterial handeln, welches vorzugsweise vollständig kristallin ist.

Es ist bevorzugt, daß die Knotenreißkraft von erfindungsgemäßem Nahtmaterial auf Basis von Polyglykolsäure innerhalb von 5 Tagen, auf weniger als 65 % der Anfangsknotenreißkraft abfällt.

Die Knotenreißkraft von erfindungsgemäßem Nahtmaterial auf Basis von Polyglykolsäure fällt innerhalb von 14 Tagen auf weniger als 10 % der Anfangsknotenreißkraft ab.

Erfindungsgemäßes Nahtmaterial auf Basis von Lactidcopolymer wird im Vergleich zu solchem auf Basis von Polyglykolsäure verzögert abgebaut. Die Knotenreißkraft fällt innerhalb von 14 Tagen auf 75 % bis 90 % der Anfangsknotenreißkraft ab.

Weiterhin ist es bevorzugt, daß die Knotenreißkraft von erfindungsgemäßem Nahtmaterial auf Basis von Lactidcopolymer innerhalb von 28 Tagen auf 60 % bis 75 % der Anfangsknotenreißkraft abfällt.

Die erwähnte Anfangsknotenreißkraft von erfindungsgemäßem Nahtmaterial liegt vorzugsweise bei mindestens 65 % der Knotenreißkraft von nicht dampfbehandeltem Nahtmaterial. Dies gilt für Nahtmaterial auf Basis von Polyglykolsäure und auch für solches, das auf einem Lactidcopolymer aufbaut.

Erfindungsgemäßes Nahtmaterial ist besonders bevorzugt durch eine thermische Behandlung von hydrolysierbarem Nahtmaterial mit Wasserdampf erhältlich. Die thermische Behandlung erfolgt vorzugsweise unter Druck. Durch die thermische Behandlung mit Wasserdampf sind vermutlich die Polymerketten des erfindungsgemäßen Nahmaterials durch Hydrolyse teilabgebaut. Dies wird bestätigt durch einen höheren Gehalt an Säuregruppen im dampfbehandelten Nahtmaterial im Vergleich zu nicht dampfbehandeltem Material. Bei Proben von dampfbehandeltem Safil® wurde unabhängig von der Fadenstärke ein Gehalt an freier Glykolsäure von vorzugsweise 20-45 mg/g, insbesondere 30 - 35 mg/g, gemessen.

Der sogenannten Knotenreißkraft kommt als Charakteristikum eines Fadens besonders große Bedeutung zu, insbesondere in der Praxis. Im Anwendungsbereich liegen Fäden meist im geknoteten Zustand vor. Der Knoten stellt aufgrund der auftretenden Scherkräfte dabei immer den schwächsten Punkt einer Naht dar. Definitionsgemäß entspricht die Knotenreißkraft der Kraft, bei der der Faden im Knoten (der aus einer festgezogenen Schlinge besteht) reißt. Unter der linearen Reißkraft versteht man dagegen die Kraft, bei der ein ungeknoteter Faden bei Zugbelastung in Richtung seiner Längsachse reißt. Die Anfangsknotenreißkraft des erfindungsgemäßen Nahtmaterials ist trotz der beschleunigten Abbaubarkeit erstaunlich hoch.

Der Abfall der erwähnten Knotenreißkraft, den ein Faden im Laufe seines Abbaus "in vivo" erfährt, ist nicht gleich zu setzen mit dem Abbau des chirurgischen Nahtmaterials. Die Funktion des Fadens bleibt nur solange bestehen, wie die Reißkraft vorhanden ist. Ein Faden ist dann vollständig abgebaut, wenn sein Material nicht mehr im Gewebe nachweisbar ist. Seine Reißkraft hat er jedoch schon lange vor diesem Zeitpunkt vollständig verloren.

In diesem Dokument vorkommende, prozentuale und absolute Angaben zur Knotenreißkraft von erfindungsgemäßem Nahtmaterial in verschiedenen Stadien sind Werte, die in einem simulierten "in vivo"-Abbau ermittelt wurden. Hierzu wurde das Nahtmaterial bei Raumtemperatur in eine Pufferlösung nach Art von Sörensen eingelegt, in bestimmten Zeitabständen (z.B. nach 5, 7, 10 und 14 Tagen) entnommen und untersucht. Das Einlegen von Nahtmaterial in eine Pufferlösung nach Art von Sörensen ist eine gängige Methode zur Simulation eines Abbaus "in vivo". Im vorliegenden Fall wurde eine wäßrige KH₂PO₄- und Na₂HPO₄ Lösung verwendet, die bei 25 °C einen pH-Wert von 7,4 aufweist. Alle in diesem Dokument vorkommenden Angaben zur Knotenreißkraft von erfindungsgemäßem Nahtmaterial nach einem Abbau "in vivo" wurden auf diese Art ermittelt. Eine weitere gängige Methode zur Simulation eines Abbaus "in vivo" besteht in der Einlagerung des Nahtmaterials in eine Kochsalzlösung (Natriumchloridlösung).

Die Anfangsknotenreißkraft von erfindungsgemäßem Nahtmaterial hängt natürlich insbesondere auch von der Fadenstärke des Nahtmaterials ab, die im folgenden gemäß der Einteilung der United States Pharmacopoe (USP) bezeichnet wird. In Tabelle 1 sind bevorzugte Wertebereiche für die Anfangsknotenreißkraft von erfindungsgemäßem Nahtmaterial auf Basis von Polyglykolsäure für unterschiedliche Fadenstärken dargestellt, jeweils im Vergleich zur Knotenreißkraft des jeweils entsprechenden Fadens vor der Behandlung nach dem erfindungsgemäßen Verfahren (Alle Angaben in Newton).

**Tabelle 1: Anfangsknotenreißkraft von erfindungsgemäßem Nahtmaterial auf Basis von Polyglykolsäure für unterschiedliche Fadenstärken, jeweils im Vergleich zur Knotenreißkraft des jeweils entsprechenden Fadens vor der Behandlung nach dem erfindungsgemäßen Verfahren.**

| Fadenstärke | Knotenreißkraft vor Dampfbehandlung [N] | Anfangsknotenreißkraft [N] (nach Dampfbehandlung) |
|---|---|---|
| USP 2 | 90-110, insbesondere 95 - 105 | 65 - 95, insbesondere 70 - 95 |
| USP 1 | 60 - 90, insbesondere 70 - 80 | 50 - 70, insbesondere 55-70 |
| USP 0 | 40 - 65, insbesondere 45 - 60 | 35 - 50, insbesondere 40 - 50 |
| USP 2-0 | 35 - 55, insbesondere 40 - 50 | 20 - 40, insbesondere 25 - 40 |
| USP 3-0 | 15 - 35, insbesondere 22 - 32 | 15 - 27, insbesondere 18-27 |
| USP 4-0 | 10 - 25, insbesondere 15-22 | 10 - 20, insbesondere 14-20 |
| USP 5-0 | 9 - 15, insbesondere 10-13 | 5 - 11, insbesondere 7 - 10 |

In einer bevorzugten Ausführungsform weist erfindungsgemäßes Nahtmaterial auf Basis von Polyglykolsäure mit einer Fadenstärke von USP 2 nach Dampfbehandlung eine Anfangsknotenreißkraft von 65 bis 95 N, insbesondere von 70 bis 95 N, auf.

In einer weiteren bevorzugten Ausführungsform weist erfindungsgemäßes Nahtmaterial auf Basis von Polyglykolsäure mit einer Fadenstärke von USP 1 nach Dampfbehandlung eine Anfangsknotenreißkraft von 50 bis 70 N, insbesondere von 55 bis 70 N, auf.

In einer weiteren insbesondere bevorzugten Ausführungsform weist erfindungsgemäßes Nahtmaterial auf Basis von Polyglykolsäure mit einer Fadenstärke von USP 0 nach Dampfbehandlung eine Anfangsknotenreißkraft von 35 bis 50 N, insbesondere von 40 bis 50 N, auf.

In einer weiteren insbesondere bevorzugten Ausführungsform weist erfindungsgemäßes Nahtmaterial auf Basis von Polyglykolsäure mit einer Fadenstärke von USP 2-0 nach Dampfbehandlung eine Anfangsknotenreißkraft von 20 bis 40 N, insbesondere von 25 bis 40 N, auf.

In einer weiteren insbesondere bevorzugten Ausführungsform weist erfindungsgemäßes Nahtmaterial auf Basis von Polyglykolsäure mit einer Fadenstärke von USP 3-0 nach Dampfbehandlung eine Anfangsknotenreißkraft von 15 bis 27 N, insbesondere von 18 bis 27 N, auf.

In einer weiteren insbesondere bevorzugten Ausführungsform weist erfindungsgemäßes Nahtmaterial auf Basis von Polyglykolsäure mit einer Fadenstärke von USP 4-0 nach Dampfbehandlung eine Anfangsknotenreißkraft von 10 bis 20 N, insbesondere von 14 bis 20 N, auf.

In einer weiteren insbesondere bevorzugten Ausführungsform weist erfindungsgemäßes Nahtmaterial auf Basis von Polyglykolsäure mit einer Fadenstärke von USP 5-0 nach Dampfbehandlung eine Anfangsknotenreißkraft von 5 bis 11 N, insbesondere von 7 bis 10 N, auf.

Über alle Fadenstärken zusammengefasst liegt der Wert für die Anfangsknotenreißkraft im Bereich zwischen 7 N und 95 N.

Nahtmaterial, das im wesentlichen aus Polyglykolsäure besteht, ist erfindungsgemäß besonders bevorzugt. In Weiterbildung ist erfindungsgemäßes Nahtmaterial aus Polyglykolsäure besonders bevorzugt, wenn es eine Viskositätszahl im Bereich zwischen 0,45 dl/g und 0,65 dl/g aufweist. Zum Vergleich, unbehandeltes Nahtmaterial aus Polyglykolsäure weist in der Regel eine Viskositätszahl im Bereich zwischen 0,9 dl/g und 1,0 dl/g auf.

Erfindungsgemäßes Nahtmaterial auf Basis von Polyglykolsäure kann ein gegenüber unbehandeltem Nahtmaterial verändertes Massenmittel seines Molekulargewichts (Dalton) aufweisen. Vorzugsweise weist das erfindungsgemäße Nahtmaterial auf Basis von Polyglykolsäure ein gegenüber unbehandeltem Nahtmaterial erniedrigtes, insbesondere um 20 bis 30 % erniedrigtes, Massenmittel seines Molekulargewichts (Dalton) auf.

Bei dem Nahtmaterial nach der Erfindung kann es sich um monofiles, multifiles, insbesondere geflochtenes monofiles, oder auch pseudomonofiles, chirurgisches Nahtmaterial handeln. Bevorzugt handelt es sich um multifiles Nahtmaterial. Grundsätzlich kann es sich auch um multifiles Nahtmaterial handeln, das aus Fäden aus unterschiedlichen Materialien gefertigt ist, z.B. aus Fäden auf Basis von Polyglykolsäure und aus Fäden auf Basis eines Lactidcopolymers.

Besonders bevorzugt weist das erfindungsgemäße Nahtmaterial eine Beschichtung auf. Beispielhaft läßt sich als bevorzugte Beschichtung eine Beschichtung aus einem Copolymer aus Glykolid, ε-Caprolacton und Trimethylencarbonat anführen. Auch Beschichtungen, die ein Salz einer Fettsäure enthalten, wie z.B. Magnesium- oder Calciumstearat, sind denkbar.

Erfindungsgemäßes Nahtmaterial ist weder in seinem Handling, noch in seinem Aussehen gegenüber unbehandeltem Nahtmaterial beeinträchtigt. Mit besonderem Vorteil ist das Verfahren auch auf mit Farbstoff versehenes Nahtmaterial (wie z.B. das eingangs erwähnte violett eingefärbte Safil®) anwendbar. Während die Farbe wie eingangs erwähnt insbesondere durch herkömmliche Behandlung mittels Gammastrahlung in Mitleidenschaft gezogen wird, tritt dieses Problem bei Behandlung im Rahmen des erfindungsgemäßen Verfahrens in deutlich geringerem Ausmaß, üblicherweise gar nicht, auf.

Des weiteren ist ein Verfahren zur Herstellung von bereits beschriebenem, erfindungsgemäßem Nahtmaterial gemäß Anspruch 9 Gegenstand dieser Erfindung Das Verfahren zeichnet sich dadurch aus, daß hydrolysierbares Nahtmaterial aus einem Polymer auf Basis von Polyglykolsäure oder Lactidcopolymer einer thermischen Behandlung mit Wasserdampf unterworfen wird.

Es ist erfindungsgemäß bevorzugt, daß die Behandlung mit Wasserdampf durchgeführt wird, der frei von Zusätzen ist, die zum oben erwähnten, teilweisen Abbau der Polymerketten des zu behandelnden Nahmaterials aktiv oder passiv beitragen können. Es ist allerdings möglich, dem Wasserdampf beispielsweise Zusätze mit ausschließlich therapeutischer Wirkung beizufügen. Besonders bevorzugt wird der Wasserdampf hingegen frei von irgendwelchen Behandlungsmitteln oder sonstigen Zusätzen in reiner, gesättigter Form verwendet.

Die thermische Behandlung im Rahmen des erfindungsgemäßen Verfahrens erfolgt bei einer Behandlungstemperatur im Bereich zwischen 120 °C und 140 °C.

Weiterhin wird die thermische Behandlung bei erhöhtem Druck im Bereich zwischen 2 bar und 5 bar durchgeführt.

Die thermische Behandlung wird über eine Dauer von 4 Minuten bis zu 30 Minuten durchgeführt. Insbesondere die kurze Behandlungsdauer stellt gegenüber herkömmlichen Verfahren, insbesondere den eingangs erwähnten Verfahren in Puffersystemen bzw. dem in einer Klimakammer, einen großen Vorteil dar.

Das behandelte Nahtmaterial wird nach der thermischen Behandlung mit Wasserdampf getrocknet. Das Trocknen des Nahtmaterials wird bei erhöhter Temperatur im Bereich zwischen 60°C und 140°C vorgenommen. Die Trocknung erfolgt unter reduziertem Druck, vorzugsweise im Bereich zwischen 0,05 bar und 0,2 bar, insbesondere im Bereich zwischen 0,05 bar und 0,1 bar. Abhängig von den Trocknungsbedingungen, insbesondere der Trocknungsdauer, können die gemessenen Werte für die Knotenreißkraft, insbesondere innerhalb von 14 Tagen, gewissen Schwankungen unterliegen. Allerdings bleibt der in den vorhergehenden Ausführungsformen der vorliegenden Erfindung beschriebene charakteristische Verlauf des Abfalls der Knotenreißkraft, insbesondere innerhalb von 14 Tagen, davon im wesentlichen unberührt.

Da die Knotenreißkraft als Charakteristikum eines Fadens insbesondere von der Stärke (Dicke bzw. Durchmesser) des Fadens abhängig ist, kann es erfindungsgemäß sinnvoll sein, die sogenannte spezifische Knotenreißkraft (Durchmesser korrigierte Knotenreißkraft, Einheit: N/mm²) zur Charakterisierung des erfindungsgemäßen Nahtmaterials zu verwenden. Die Charakterisierung des erfindungsgemäßen Nahtmaterials durch seine spezifische Knotenreißkraft ergibt, daß das Nahtmaterial eine im Vergleich zu seinem Abbau hohe spezifische Anfangskontenreißkraft (Durchmesser korrigierte Anfangsknotenreißkraft, Einheit: N/mm²) besitzt, und die spezifische Knotenreißkraft, insbesondere innerhalb von 14 Tagen, vorzugsweise schnell abfällt, wobei der Abfall der Knotenreißkraft zumindest teilweise linear verläuft. Die Charakterisierung durch die spezifische Knotenreißkraft ermöglicht insbesondere einen Vergleich mit anderen Nahtmaterialien, insbesondere in verschiedenen Fadenstärken.

Mit besonderem Vorteil weist das erfindungsgemäße Nahtmaterial, insbesondere Nahtmaterial auf Basis von Polyglykolsäure, eine im Vergleich zu einem entsprechenden Nahtmaterial, welches durch herkömmliche Sterilisationsverfahren, insbesondere Bestrahlung, behandelt ist, geringere spezifische Knotenreißkraft auf. Bevorzugt weist das erfindungsgemäße Nahtmaterial eine geringere spezifische Knotenreißkraft auf als ein entsprechend mit Gammabestrahlung (γ-Bestrahlung) behandeltes Nahtmaterial, wobei für die Bestrahlung übliche Strahlendosen, insbesondere in einem Bereich zwischen 30 und 70 kGy (kilo Gray), vorzugsweise eine Strahlendosis von ca. 32 kGy oder ca. 64 kGy, verwendet werden. Zwar läßt sich die spezifische Knotenreißkraft von Nahtmaterialien durch höhere Strahlendosen grundsätzlich verringern, jedoch sind der Bestrahlung von Nahtmaterialien wegen ihrer Verpackung Grenzen gesetzt. Herkömmliche Verpackungsmaterialien können einer Strahlendosis von maximal ca. 70 kGy ausgesetzt werden.

Weiterhin kann es erfindungsgemäß sinnvoll sein, das Nahtmaterial durch seine sogenannte spezifische Anfangsknotenreißkraft zu charakterisieren. Das erfindungsgemäße Nahtmaterial auf Basis von Polyglykolsäure, insbesondere Safil®, weist vorzugsweise eine gegenüber einem Nahtmaterial auf Basis von Polyglykolsäure und Milchsäure, beispielsweise Vicryl® und Vicryl® Rapid, höhere spezifische Anfangsknotenreißkraft auf.

Die Durchführung der thermischen Behandlung mit Wasserdampf erfolgt in einem Autoklaven. Vorzugsweise werden die zu behandelnden Fäden in diesem sowohl dampfbehandelt als auch anschließend getrocknet. Die Temperatur im Autoklaven ist bevorzugt, wie auch der Druck, regelbar. Der Autoklav weist vorzugsweise eine ausreichende Größe auf, um auch eine größere Menge an Nahtmaterial aufnehmen zu können. Es ist bevorzugt, daß der zur Durchführung des erfindungsgemäßen Verfahrens benötigte Wasserdampf unmittelbar zugeführt werden kann. Alternativ ist es aber auch denkbar, den Wasserdampf durch Verdampfung im Autoklaven zu erzeugen.

Die thermische Behandlung mit Wasserdampf wird im wesentlichen frei von Luft vorgenommen. Sie erfolgt in einer reinen Wasserdampfatmosphäre. Dies wird realisiert, indem der Autoklav vor der Dampfbehandlung evakuiert und gegebenenfalls auch mit einem inerten Gas oder einem anderen geeigneten Fluid gespült wird.

Mit besonderem Vorteil ist durch das erfindungsgemäße Verfahren die anfängliche Knotenreißkraft des hergestellten resorbierbaren chirurgischen Nahtmaterials in Abhängigkeit von der Fadenstärke einstellbar. Diese Einstellung erfolgt vorzugsweise durch geeignete Wahl der Behandlungsdauer, wodurch der Anteil an teilabgebauten Polymerketten im behandelten Nahtmaterial beeinflußbar ist. Grundsätzlich ist die anfängliche Knotenreißkraft des hergestellten resorbierbaren chirurgischen Nahtmaterials aber auch durch Variation der Parameter Druck und Temperatur oder auch durch gleichzeitige Variation von mindestens zwei der genannten Parameter zu beeinflussen.

Bei der Behandlung von hydrolysierbarem Nahtmaterial nach dem erfindungsgemäßen Verfahren wird Nahtmaterial erhalten, dessen Knotenreißkraft etwas geringer ist, als die des Ausgangsmaterials. Üblich sind Abnahmen der Knotenreißkraft durch die Behandlung von etwa 10%. Abhängig von Material, Beschaffenheit, Durchmesser des Nahtmaterials vor der Behandlung und gegebenenfalls weiteren Parametern sind Abweichungen von diesem Wert möglich.

Des weiteren wurde festgestellt, daß beim erfindungsgemäßen Verfahren eine Längenschrumpfung des behandelten Nahtmaterials auftritt wenn sie nicht verhindert wird, beispielsweise durch Einspannen der Fäden. Gegebenenfalls tritt diese in Kombination mit einer Zunahme des Durchmessers des behandelten Nahtmaterials auf. Auch die Längenschrumpfung ist gegebenenfalls abhängig von der Wahl der Ausgangsmaterialien, insbesondere auch deren Beschaffenheit und Durchmesser. Das resultierende Nahtmaterial kann um bis zu 40% kürzer als das Ausgangsmaterial sein. Gewöhnlich beträgt die Schrumpfung nicht weniger als 2,5 %.

Vorzugsweise wird durch das erfindungsgemäße Verfahren im wesentlichen aus Polyglykolsäure bestehendes Nahtmaterial behandelt. Bei im wesentlichen aus Polyglykolsäure bestehendem Nahtmaterial wurde beispielsweise bei einer Dampfbehandlung von Safil® violett USP 5-0 über 5 min bei 121 °C ein Durchmesserzuwachs um etwa 7 % beobachtet.

Bei einer anderen Ausführungsform wird durch das erfindungsgemäße Verfahren im wesentlichen aus Lactidcopolymer bestehendes Nahtmaterial behandelt. Der erwähnte Durchmesserzuwachs kann hier in einigen Fällen, wie z.B. bei einem in der EP 999227 A2 beschriebenen Lactidcopolymer aus 75 % Hart- und 25 % Weichanteil, sogar bis zu 63 % betragen. Im speziellen Falle dieses Blockcopolymers aus einer Weich-Hart-Kombination setzte sich das Hartsegment aus 95 Gew.-% L-Lactid, und 5 Gew.-% Trimethylencarbonat (TMC) zusammen, das Weichsegment aus 45 Gew.-% ε-Caprolacton, 45 Gew.-% Trimethylencarbonat und 10 Gew.-% L-Lactid.

Durch das erfindungsgemäße Verfahren läßt sich multifiles, monofiles und auch pseudomonofiles Nahtmaterial behandeln. Grundsätzlich ist die diesbezügliche Beschaffenheit des Nahtmaterials im wesentlichen unkritisch.

Durch das erfindungsgemäße Verfahren kann auch Nahtmaterial behandelt werden, das eine Beschichtung aufweist, insbesondere eine Beschichtung aus einem Copolymer aus Glykolid, ε-Caprolacton und Trimethylencarbonat. Grundsätzlich ist es denkbar, durch entsprechende Auswahl der Beschichtung auf das Verfahren Einfluß zu nehmen und das Abbauverhalten von nach dem erfindungsgemäßen Verfahren behandeltem Nahtmaterial zu beeinflussen.

Auch eingefärbtes Nahtmaterial ist durch das erfindungsgemäße Verfahren behandelbar, wie bereits an anderer Stelle erwähnt wurde.

In einer weiteren bevorzugten Ausführungsform wird durch das erfindungsgemäße Verfahren gebrauchsfertiges Nahtmaterial behandelt, also insbesondere auch Nahtmaterial, das bereits mit einer Nadel versehen ist. Im Gegensatz zu manchen anderen Verfahren ist das erfindungsgemäße Verfahren universell anwendbar, es läßt sich nämlich sowohl in den Produktionsprozeß integrieren als auch, wie hier hervorgehoben, auf gebrauchsfertiges Nahtmaterial anwenden.

Auch alle Nahtmaterialien, die nach einem erfindungsgemäßen Verfahren hergestellt bzw. herstellbar sind, sind Gegenstand dieser Erfindung.

Ein nach dem erfindungsgemäßen Verfahren behandelter Faden läßt sich grundsätzlich sehr lange lagern.

Weitere Einzelheiten und Merkmale der Erfindung ergeben sich aus der nachfolgenden Beschreibung bevorzugter Ausführungsformen in Kombination mit den Zeichnungen, den Beispielen und den Unteransprüchen. In diesen Ausführungsformen können einzelne Merkmale der Er findung allein oder in Kombination mit anderen Merkmalen verwirklicht sein. Die beschriebenen bevorzugten Ausführungsformen sind lediglich als beschreibende, keineswegs als in irgendeiner Weise limitierende Offenbarung zu verstehen. Sämtliche Zeichnungen werden hiermit durch ausdrückliche Bezugnahme zum Inhalt dieser Beschreibung gemacht.

Die Zeichnungen zeigen:
- In Abb. 1 einen für eine Ausführungsform des erfindungsgemäßen Verfahrens typischer Dampfzyklus.
- In Abb. 2 den Abfall der Knotenreißkraft eines monofilen Nahtmaterials aus einem Lactidcopolymer nach Dampfbehandlung bei 121 °C über einen Zeitraum von 5 min. im Vergleich zu unbehandeltem Nahtmaterial.
- In Abb. 3 den Abfall der Knotenreißkraft von Safil® violett USP 2 nach Dampfbehandlung bei unterschiedlichen Temperaturen.
- In Abb. 4 den Abfall der Knotenreißkraft von Safil® violett USP 1 nach Dampfbehandlung bei unterschiedlichen Temperaturen.
- In Abb. 5 den Abfall der Knotenreißkraft von Safil® violett USP 0 nach Dampfbehandlung bei unterschiedlichen Temperaturen.
- In Abb. 6 den Abfall der Knotenreißkraft von Safil® violett USP 2-0 nach Dampfbehandlung bei unterschiedlichen Temperaturen.
- In Abb. 7 den Abfall der Knotenreißkraft von Safil® violett USP 3-0 nach Dampfbehandlung bei unterschiedlichen Temperaturen.
- In Abb. 8 den Abfall der Knotenreißkraft von Safil® violett USP 4-0 nach Dampfbehandlung bei unterschiedlichen Temperaturen.
- In Abb. 9 den Abfall der Knotenreißkraft von Safil® violett USP 5-0 nach Dampfbehandlung bei unterschiedlichen Temperaturen.
- In Abb. 10 die anfängliche Knotenreißkraft von erfindungsgemäß behandeltem Nahtmaterial im Vergleich zu unbehandelten Nahtmaterial sowie zu weiteren bekannten resorbierbaren Nahtmaterialien sowie den Abfall der Knotenreißkraft im Vergleich.
- In Abb. 11 den relativen Durchmesserzuwachs dampfbehandelter Safil®-Proben im Vergleich zu unbehandeltem Safil®.
- In Abb. 12 die lineare Reißkraft von Polysorb® 3-0 und Safil® 3-0 nach Behandlung bei 54,4 °C bei 50 % relativer Luftfeuchtigkeit und das daraus resultierende Abbauverhalten "in vivo" bzw. den Rückgang der Knotenreißkraft bei Einlagerung in eine Pufferlösung mit einem pH-Wert von 7,4 nach Art von Sörensen.

### Beispiel

Eine besonders bevorzugte Ausführungsform des erfindungsgemäßen Verfahrens wird im folgenden beschrieben:

Ein oder mehrere der zu behandelnden Fäden werden auf ein Sieb gelegt und zur Durchführung des erfindungsgemäßen Verfahrens in den Innenraum eines Autoklaven überführt. Bei den Fäden handelt es sich um handelsübliche Fäden aus Polyglykolsäure (z.B. Safil®). Nach Einbringen des zu behandelnden Nahtmaterials wird der Autoklav geschlossen und zunächst evakuiert. Vorzugsweise ist der Autoklav zu diesem Zeitpunkt bereits beheizt, im vorliegenden Fall auf 121 °C. Gegebenenfalls kann die Evakuierung wiederholt werden, auch verbunden mit einem Spülen des Autoklaven mit einem geeigneten Fluid. Nach einmaligem Evakuieren wird der Autoklav mit Wasserdampf befüllt bis er seinen Enddruck (ca. 2.100 Millibar) erreicht hat. Der erreichte Druck wird über ca. 10 Minuten konstant gehalten. Die Temperatur im Autoklaven beträgt dabei konstant 121 °C. Danach erfolgt eine zweite Evakuierung des Autoklaven innerhalb von ca. 60 Sekunden. Die Evakuierung wird über ca. 8 min. aufrechterhalten, in diesem Zeitraum trocknet das Nahtmaterial bei unveränderter Temperatur. Nach abgeschlossener Trocknung ist die Behandlung gemäß dem erfindungsgemäßen Verfahren beendet. Der Autoklav wird belüftet und geöffnet und das behandelte Nahtmaterial wird entnommen.

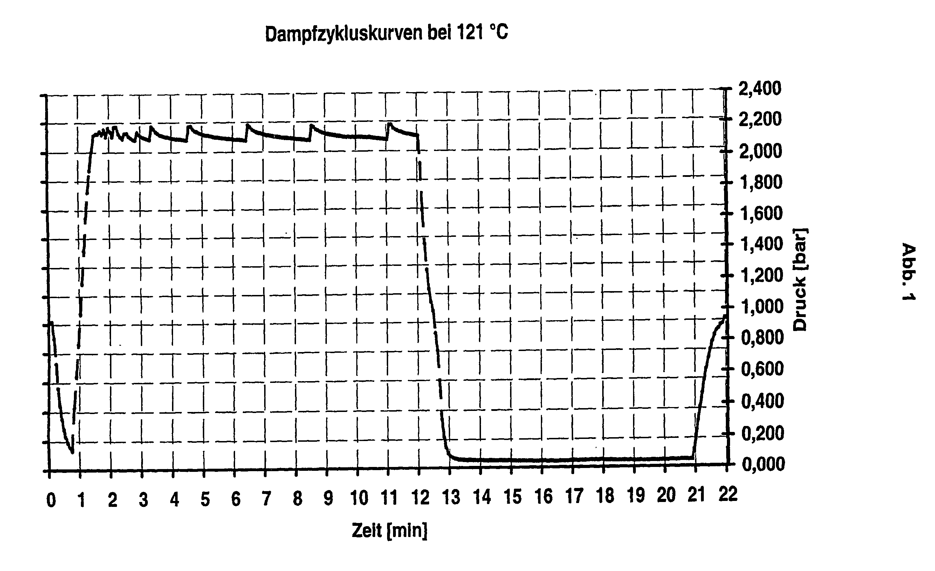

Der Dampfzyklus für diese Ausführungsform des erfindungsgemäßen Verfahrens bei 121 °C ist in Abbildung 1 schematisch (Druck in mbar aufgetragen gegen Zeit in min) aufgezeigt.

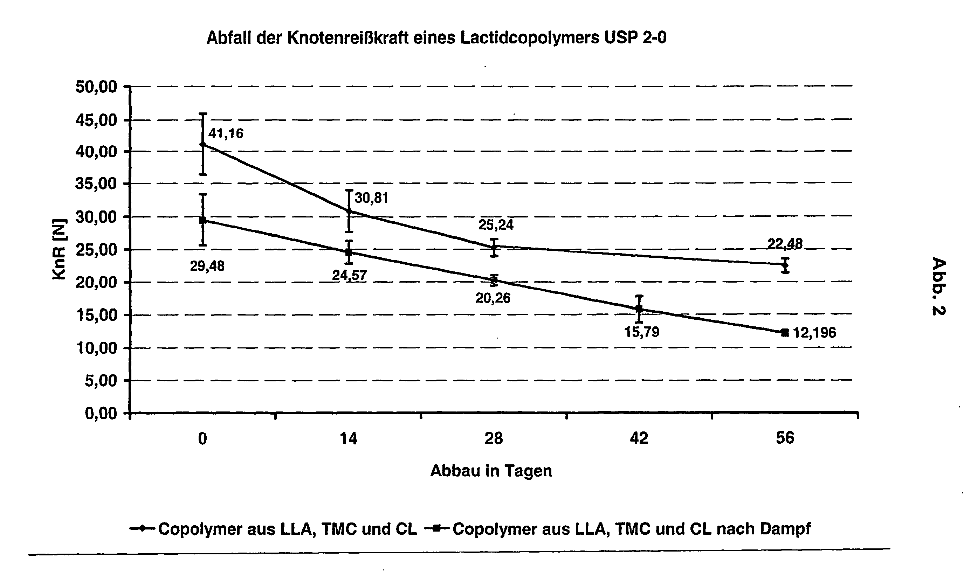

Anhand von Abbildung 2 wird der mit einem Abbau "in vivo" verbundene Rückgang der Knotenreißkraft von nach dem erfindungsgemäßen Verfahren behandelten Nahtmaterial aus einem Lactidcopolymer (USP 2-0), wie es in der EP 999227 A2 beschrieben ist, illustriert. Das Nahtmaterial wurde nach erfindungsgemäßer Behandlung "in vitro"-Bedingungen ausgesetzt, die einen Abbau "in vivo" simulieren, wie bereits an anderer Stelle beschrieben wurde. Zuvor wurde die Anfangsknotenreißkraft (nach Behandlung, aber vor dem Abbau, also zum Zeitpunkt 0 in Abb. 2) bestimmt, im Verlauf des Abbaus dann die verbleibende Knotenreißkraft nach 14, 28, 42 und 56 Tagen Einlagerung bei Raumtemperatur in eine Pufferlösung mit pH = 7,4 nach Art von Sörensen wie weiter oben bereits beschrieben. Die obere Kurve im Schaubild illustriert das Abbauverhalten von unbehandeltem Lactidcopolymer, die untere Kurve das von erfindungsgemäß behandeltem. Das Nahtmaterial gemäß der EP 999227 A2 setzt sich aus einem Blockcopolymer aus 75 % Hart- und 25 % Weichanteil zusammen, wobei das Hartsegment aus 95 Gew.-% L-Lactid (LLA) und 5 Gew.-% Trimethylencarbonat (TMC) besteht und das Weichsegment aus 45 Gew.-% ε-Caprolacton (CL), 45 Gew.-% Trimethylencarbonat und 10 Gew.-% L-Lactid. Der Abbildung ist zu entnehmen, daß die Knotenreißkraft des untersuchten dampfbehandelten Nahtmaterials unter "in vivo"-Bedingungen über einen Zeitraum von 56 Tagen im wesentlichen linear abfällt.

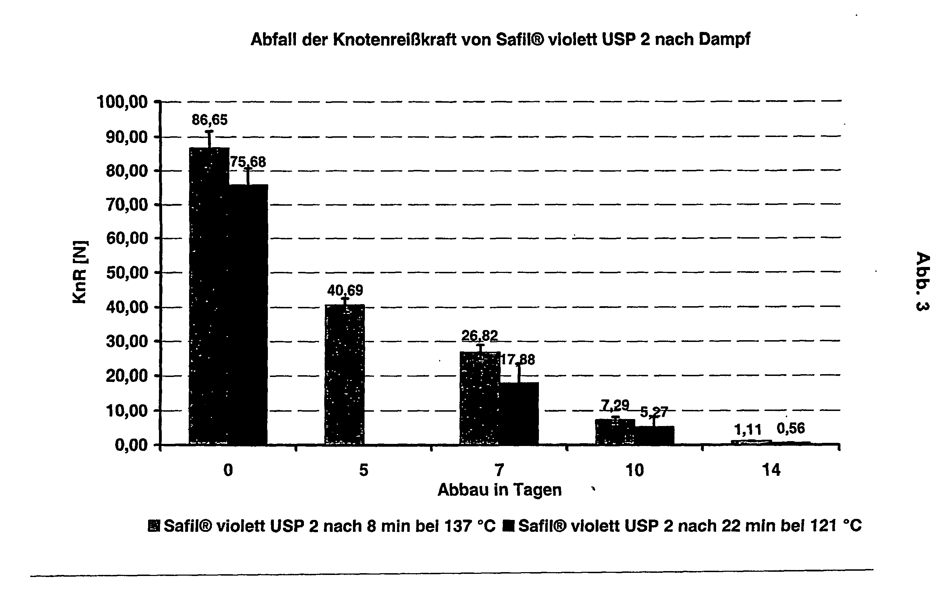

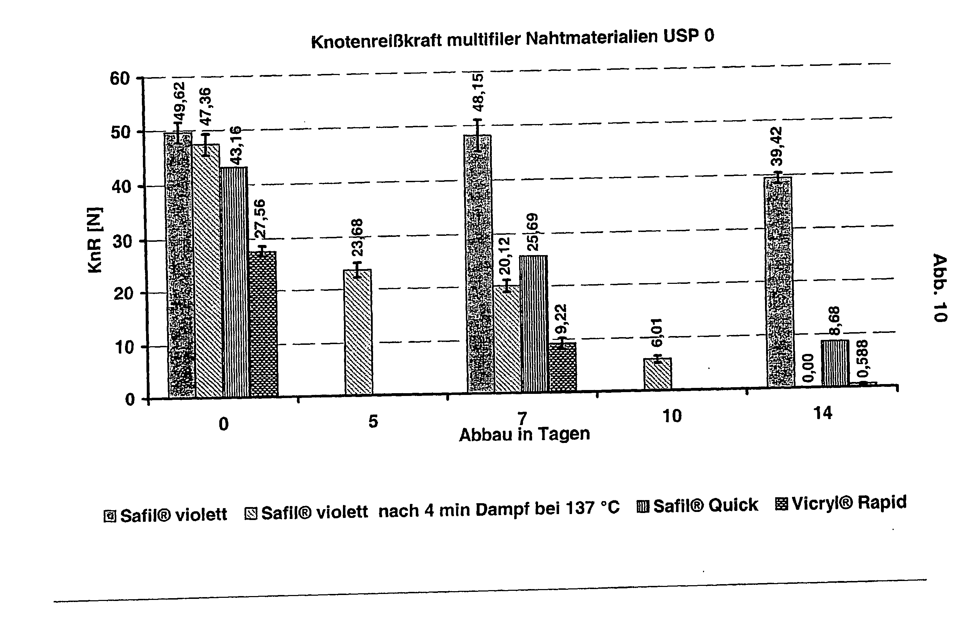

Auch in den Abbildungen 3 bis 9 wird der der mit einem Abbau "in vivo" verbundene Rückgang der Knotenreißkraft von erfindungsgemäß behandeltem Nahtmaterial illustriert, allerdings von Nahtmaterial auf Basis von Polyglykolsäure. In Abbildung 10 wird das Abbauverhalten von erfindungsgemäß behandeltem Nahtmaterial mit dem von anderen Nahtmaterialien verglichen. Das Nahtmaterial wurde in allen Fällen den bereits genannten "in vitro" Bedingungen ausgesetzt. Es wurde die anfängliche Knotenreißkraft bestimmt sowie die verbleibende Knotenreißkraft nach einem Abbau von 5, 7, 10 und 14 Tagen Einlagerung bei Raumtemperatur in Pufferlösung mit einem pH-Wert von 7,4 nach Art von Sörensen. Die Dampfbehandlungen des untersuchten Nahtmaterials waren durchgeführt worden wie oben im Beispiel beschrieben. Bei 121 °C entsprach die Dampfdruckkurve in allen Fällen im wesentlichen der in Abb. 1 beschriebenen. Bei Dampfbehandlungen bei 137 °C verschiebt sich die im Autoklaven gemessene Dampdruckkurve entsprechend der Dampfdruckkurve von Wasser. Messungen ergaben bei Dampfbehandlungen bei 137 °C einen Enddruck von ca. 3,3 bar.

So zeigt Abb. 3 den Abfall der Knotenreißkraft von Safil® violett USP 2 (für dessen Knotenreißkraft vor der Dampfbehandlung ein Wert von 97,8 N ermittelt wurde) nach 8 min. Dampfbehandlung bei 137 °C und nach 22 min. Dampfbehandlung bei 121 °C. Die jeweils rechts angeordneten, dunkel dargestellten Balken beschreiben die Knotenreißkraft des bei 121 °C behandelten Nahtmaterials, die jeweils links angeordneten, heller dargestellten Balken die des bei 137 °C behandelten.

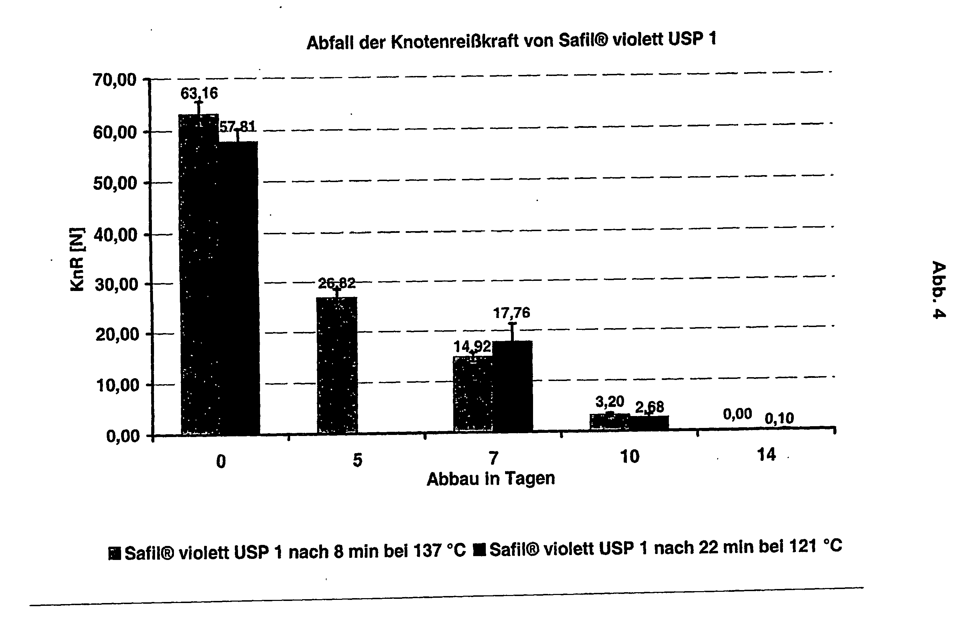

Abb. 4 zeigt den Abfall der Knotenreißkraft von Safil® violett USP 1 (für dessen Knotenreißkraft vor der Dampfbehandlung ein Wert von 74,25 N ermittelt wurde) nach 8 min. Dampfbehandlung bei 137 °C und nach 22 min. Dampfbehandlung bei 121 °C. Die jeweils rechts angeordneten, dunkel dargestellten Balken beschreiben auch hier die Knotenreißkraft des bei 121 °C behandelten Nahtmaterials, die jeweils links angeordneten, heller dargestellten Balken die des bei 137 °C behandelten.

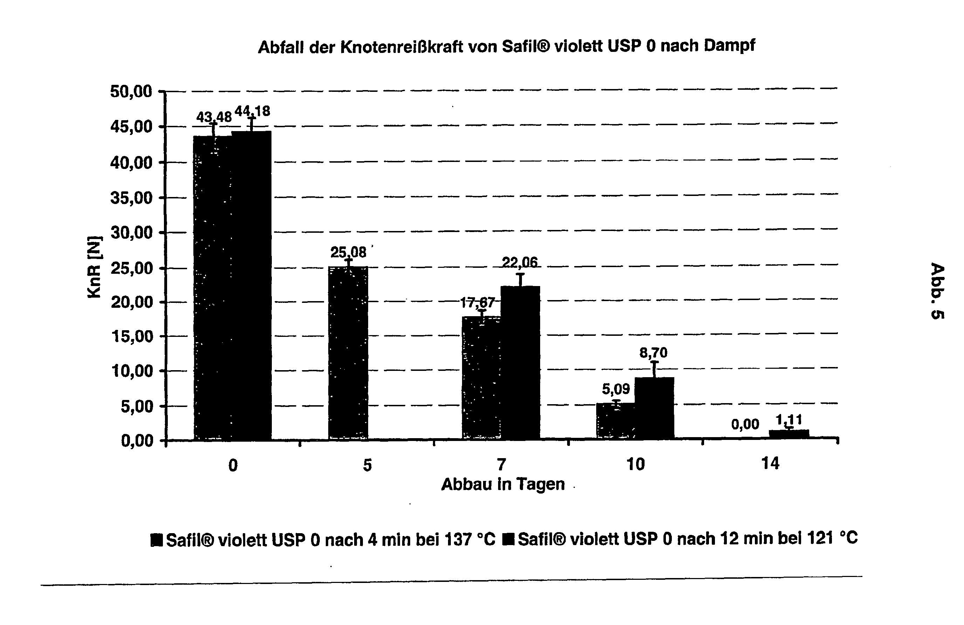

Abb. 5 zeigt den Abfall der Knotenreißkraft von Safil® violett USP 0 (für dessen Knotenreißkraft vor der Dampfbehandlung ein Wert von 52,85 N ermittelt wurde) nach 4 min. Dampfbehandlung bei 137 °C und nach 12 min. bei 121 °C. Die jeweils rechts angeordneten, dunkel dargestellten Balken beschreiben wiederum die Knotenreißkraft des bei 121 °C behandelten Nahtmaterials, die jeweils links angeordneten, heller dargestellten Balken die des bei 137 °C behandelten.

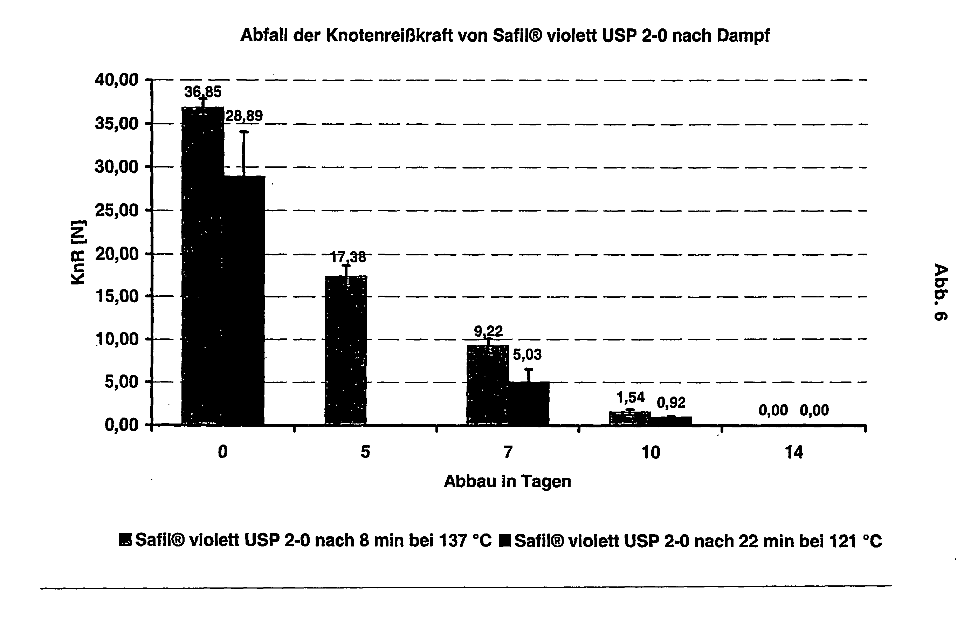

Abb. 6 zeigt den Abfall der Knotenreißkraft von Safil® violett USP 2-0 (für dessen Knotenreißkraft vor der Dampfbehandlung ein Wert von 44,80 N ermittelt wurde) nach 8 min Dampfbehandlung bei 137 °C und nach 22 min Dampfbehandlung bei 121 °C. Die jeweils rechts angeordneten, dunkel dargestellten Balken beschreiben auch hier die Knotenreißkraft des bei 121 °C behandelten Nahtmaterials, die jeweils links angeordneten, heller dargestellten Balken die des bei 137 °C behandelten.

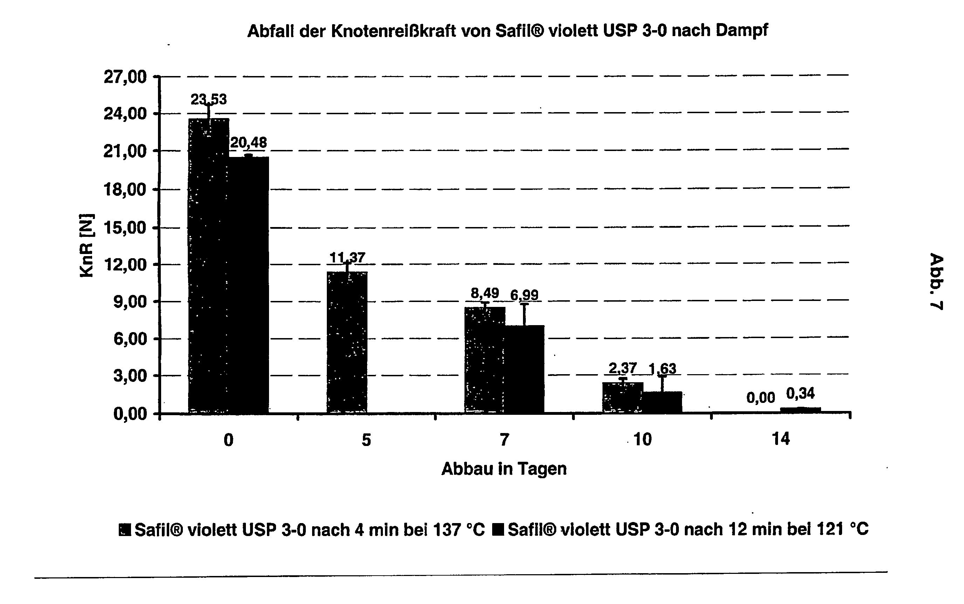

Abb. 7 zeigt den Abfall der Knotenreißkraft von Safil® violett USP 3-0 (für dessen Knotenreißkraft vor der Dampfbehandlung ein Wert von 27,11 N ermittelt wurde) nach 4 min Dampfbehandlung bei 137 °C und nach 12 min. Dampfbehandlung bei 121 °C. Die jeweils rechts angeordneten, dunkel dargestellten Balken beschreiben auch hier die Knotenreiß®kraft des bei 121 °C behandelten Nahtmaterials, die jeweils links angeordneten, heller dargestellten Balken die des bei 137 °C behandelten.

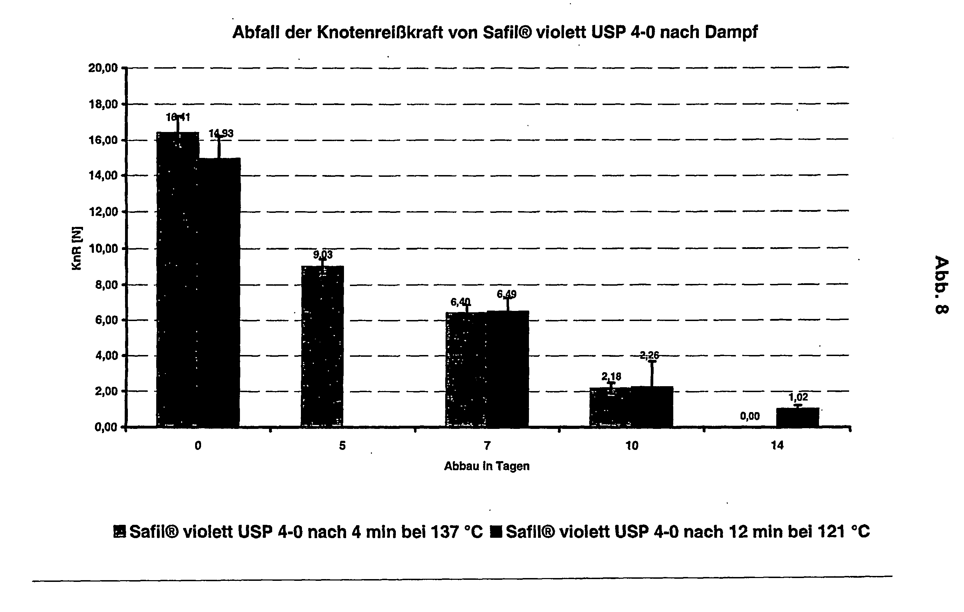

Abb. 8 zeigt den Abfall der Knotenreißkraft von Safil® violett USP 4-0 (für dessen Knotenreißkraft vor der Dampfbehandlung ein Wert von 18,55 N ermittelt wurde) nach 4 min Dampfbehandlung bei 137 °C und nach 12 min. Dampfbehandlung bei 121 °C. Die jeweils rechts angeordneten, dunkel dargestellten Balken beschreiben auch hier die Knotenreißkraft des bei 121 °C behandelten Nahtmaterials, die jeweils links angeordneten, heller dargestellten Balken die des bei 137 °C behandelten.

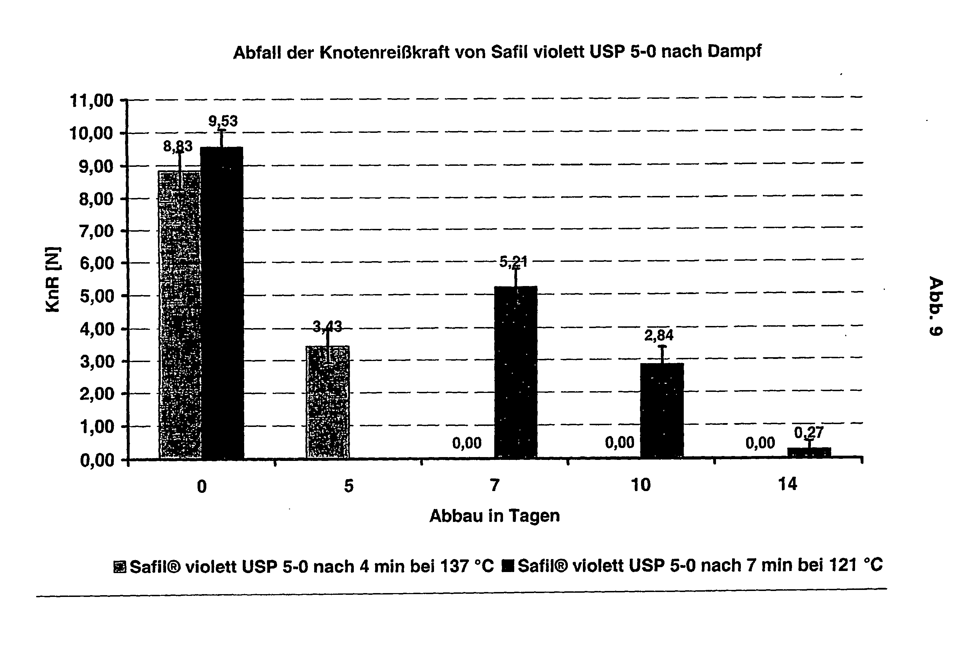

Abb. 9 zeigt den Abfall der Knotenreißkraft von Safil®violett USP 5-0 (für dessen Knotenreißkraft vor der Dampfbehandlung ein Wert von ca. 12 N ermittelt wurde) nach 4 min Dampfbehandlung bei 137 °C und nach 7 min. Dampfbehandlung bei 121 °C. Die jeweils rechts angeordneten, dunkel dargestellten Balken beschreiben auch hier die Knotenreißkraft des bei 121 °C behandelten Nahtmaterials, die jeweils links angeordneten, heller dargestellten Balken die des bei 137 °C behandelten. Es fällt auf, daß im Fall des erfindungsgemäß behandelten Safil® violett USP 5-0 die Knotenreißkraft schon nach 7 Tagen Abbau auf nicht mehr messbare Werte abgefallen ist.

Abb. 10 zeigt die Anfangsknotenreißkraft von erfindungsgemäß behandeltem Nahtmaterial (Safil® violett A60 USP 0 behandelt mit Dampf bei 137 °C über eine Dauer von 4 min) im Vergleich zu unbehandelten Nahtmaterial (Safil® violett A60 USP 0) sowie zu den zwei unter den Handelsbezeichnungen "Safil® Quick" und "Vicryl® Rapid" bekannten Nahtmaterialien (beide ebenfalls USP 0) sowie den Abfall der Knotenreißkraft im Vergleich. Bei Safil® Quick handelt es sich um von der Anmelderin vertriebenes, chirurgisches Nahtmaterial mit beschleunigter Resorbierbarkeit. Die Knotenreißkraft von unbehandeltem Safil® violett ist auf der Zeitachse jeweils als dunkelgrauer, weiß gepunkteter Balken dargestellt (bei 0 Tagen ganz links). Der hellere, schräg schraffierte Balken unmittelbar rechts davon illustriert jeweils die Knotenreißkraft von erfindungsgemäß behandeltem Nahtmaterial. Die Knotenreißkraft von Safil® Quick wird jeweils durch den längsgestreiften Balken (bei 0 Tagen dritter von links) dargestellt, während die von Vicryl® Rapid durch den bei 0 Tagen ganz rechts angeordneten Balken illustriert ist. Dem Diagramm ist zu entnehmen, dass die Knotenreißkraft von erfindungsgemäß behandeltem Nahtmaterial deutlich schneller abfällt als die von unbehandeltem. Während z.B. das unbehandelte Safil® violett nach 14 Tagen noch ca. 80 % seiner ursprünglichen Knotenreißkraft aufweist ist die Knotenreißkraft von behandeltem Safil®violett nach 14 Tagen nicht mehr messbar.

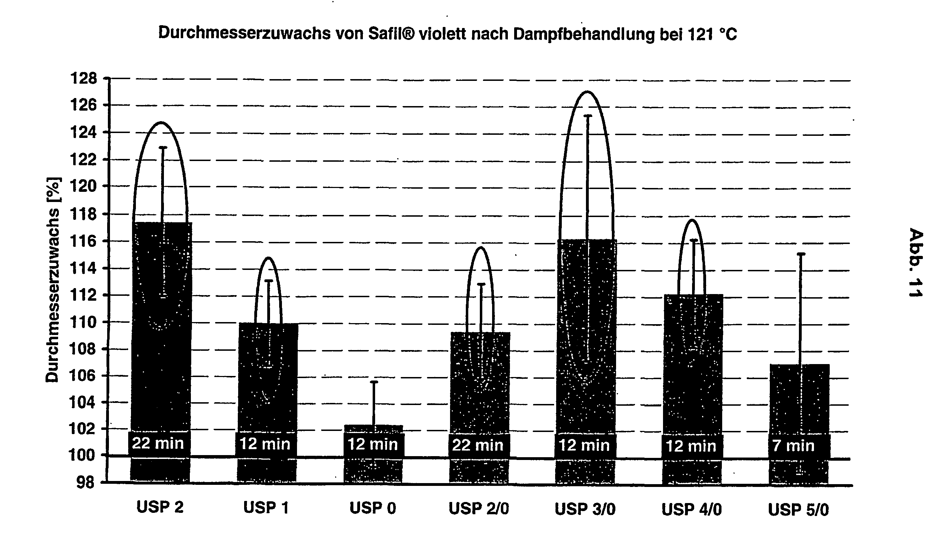

Abb. 11 illustriert den Durchmesserzuwachs, dem Nahtmaterial bei Behandlung nach dem erfindungsgemäßen Verfahren unterliegen kann. Gezeigt ist die jeweilige prozentuale Zunahme des Durchmessers von Safil®-Proben verschiedener Fadenstärken, die sich aus einer Dampfbehandlung der Proben nach dem erfindungsgemäßen Verfahren ergeben kann.

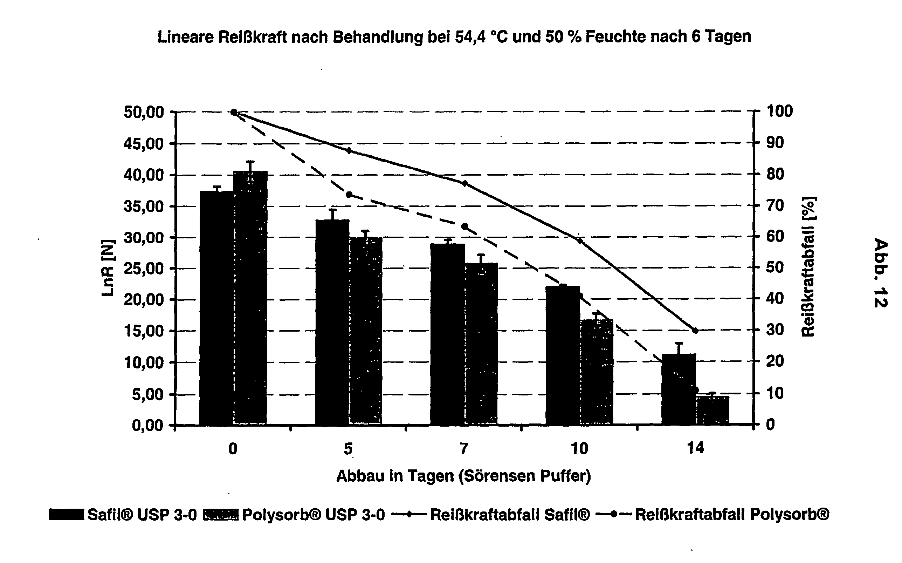

Abb. 12 zeigt die anfängliche lineare Reißkraft von Polysorb® 3-0 und Safil® 3-0, in beiden Fällen nach Behandlung in einer Klimakammer gemäß der eingangs genannten WO 2004/050127 bei 54,4 °C bei 50 % relativer Luftfeuchtigkeit. Weiterhin ist der Abbildung das daraus resultierende "in vivo"-Abbauverhalten zu entnehmen, bzw. der Rückgang der Knotenreißkraft bei Einlagerung in Pufferlösung mit pH = 7,4 nach Art von Sörensen. Die beiden untersuchten Nahtmaterialien zeigen ein ähnliches Abbauverhalten. Die Knotenreißkraft fällt anfangs im wesentlichen gleichmäßig und vergleichsweise langsam ab, während gegen Ende der Abfall der Knotenreißkraft beschleunigt ist. Dies steht im Gegensatz zu einem Abbauverhalten, wie es sich insbesondere aus den Abbildungen 3 bis 8 entnehmen läßt. Dort fällt die Knotenreißkraft, ausgehend von einem relativ hohen Ausgangswert, anfänglich vergleichsweise schnell ab, wohingegen der Abfall mit fortschreitender Zeit verlangsamt ist.

## Patentansprüche

1. Resorbierbares, thermisch dampfbehandeltes, chirurgisches Nahtmaterial auf Basis von Polyglykolsäure oder Lactidcopolymer mit einem schnellen Abbau und einem schnellen Abfall der Knotenreißkraft unter "in vivo"-Bedingungen und einer im Verhältnis zum schnellen Abbau hohen Anfangsknotenreißkraft, **dadurch gekennzeichnet, daß** die Anfangsknotenreißkraft im Bereich zwischen 7 N und 95 N liegt und die Knotenreißkraft des Nahtmaterials auf Basis von Polyglykolsäure innerhalb von 14 Tagen auf weniger als 10 % der Anfangsknotenreißkraft und die Knotenreißkraft des Nahtmaterials auf Basis von Lactidcopolymer innerhalb von 14 Tagen auf 75 % bis 90 % der Anfangsknotenreißkraft abfällt.

2. Nahtmaterial auf Basis von Polyglykolsäure nach Anspruch 1, **dadurch gekennzeichnet, daß** die Knotenreißkraft innerhalb von 5 Tagen auf weniger als 65 % der Anfangsknotenreißkraft abfällt.

3. Nahtmaterial auf Basis von Lactidcopolymer nach Anspruch 1, **dadurch gekennzeichnet, daß** die Knotenreißkraft innerhalb von 28 Tagen auf 60 % bis 75 % der Anfangsknotenreißkraft abfällt.

4. Nahtmaterial nach einem der vorhergehenden Ansprüche, **dadurch gekennzeichnet, daß** die Anfangsknotenreißkraft mindestens 65 % der Knotenreißkraft von nicht dampfbehandeltem Nahtmaterial beträgt.

5. Nahtmaterial nach einem der vorhergehenden Ansprüche, erhältlich durch thermische Behandlung von hydrolisierbarem Nahtmaterial mit Wasserdampf.

6. Nahtmaterial nach Anspruch 5, **dadurch gekennzeichnet, daß** die thermische Behandlung eine Druckdampfbehandlung ist.

7. Nahtmaterial nach einem der vorhergehenden Ansprüche, **dadurch gekennzeichnet, daß** die Anfangsknotenreißkraft zwischen 10 N und 90 N beträgt.

8. Nahtmaterial auf Basis von Polyglykolsäure nach einem der Ansprüche 1 oder 2 oder 4 bis 7, **dadurch gekennzeichnet, daß** es eine Viskositätszahl von 0.45 - 0.65 dl/g aufweist.

9. Verfahren zur Herstellung von schnell resorbierbarem, chirurgischen Nahtmaterial gemäß einem der Ansprüche 1 bis 8, **dadurch gekennzeichnet, daß** hydrolisierbares Nahtmaterial auf Basis von Polyglykolsäure oder Lactidcopolymer einer thermischen Behandlung mit Wasserdampf unterworfen wird, die thermische Behandlung mit Wasserdampf in einem Autoklaven und im wesentlichen frei von Luft vorgenommen wird, der Autoklav vor der Dampfbehandlung evakuiert wird, die thermische Behandlung bei einer Behandlungstemperatur zwischen 120 °C und 140 °C durchgeführt, bei einem Druck zwischen 2 und 5 bar durchgeführt, über eine Behandlungsdauer von 4 min bis 30 min vorgenommen und das Nahtmaterial nach der thermischen Behandlung zwischen 60 °C und 140 °C getrocknet und die Trocknung unter reduziertem Druck durchgeführt wird.

10. Verfahren nach Anspruch 9, **dadurch gekennzeichnet, daß** die Behandlung mit Wasserdampf durchgeführt wird, der frei von Zusätzen ist.

11. Verfahren nach einem der Ansprüche 9 bis 10, **dadurch gekennzeichnet, daß** die thermische Behandlung bei einem Druck zwischen 2 bar und 3 bar durchgeführt wird.

12. Verfahren nach einem der Ansprüche 9 bis 11, **dadurch gekennzeichnet, daß** die thermische Behandlung über eine Behandlungsdauer von 4 min bis 22 min vorgenommen wird.

13. Verfahren nach Anspruch 9, **dadurch gekennzeichnet, daß** die Trocknung zwischen 0,05 bar und 0,2 bar durchgeführt wird.

14. Verfahren nach einem der Ansprüche 9 bis 13, **dadurch gekennzeichnet, daß** die thermische Behandlung mit Wasserdampf in einer reinen Wasserdampfatmosphäre vorgenommen wird.

15. Verfahren nach einem der Ansprüche 9 bis 14, **dadurch gekennzeichnet, daß** gebrauchsfertiges Nahtmaterial behandelt wird, insbesondere bereits mit einer Nadel versehenes Nahtmaterial.

16. Nahtmaterial, herstellbar nach einem Verfahren nach einem der Ansprüche 9 bis 15.

## Claims

1. Absorbable, thermally steam-treated, surgical suture based on polyglycolic acid or lactide copolymer comprising rapid degradation and rapid decrease in knot-pull breaking strength under in vivo conditions and an initial knot-pull breaking strength which is high for the rapid degradation, **characterized in that** the initial knot-pull breaking strength is in the range between 7 N and 95 N and the knot-pull breaking strength of the suture based on polyglycolic acid decreases within 14 days to less than 10% of the initial knot-pull breaking strength and the knot-pull breaking strength of the suture based on lactide copolymer decreases within 14 days to 75% to 90% of the initial knot-pull breaking strength.

2. Suture based on polyglycolic acid according to Claim 1, **characterized in that** the knot-pull breaking strength decreases within 5 days to less than 65% of the initial knot-pull breaking strength.

3. Suture based on lactide copolymer according to Claim 1, **characterized in that** the knot-pull breaking strength decreases within 28 days to 60% to 75% of the initial knot-pull breaking strength.

4. Suture according to any one of the preceding claims, **characterized in that** the initial knot-pull breaking strength is at least 65% of the knot-pull breaking strength of non-steam-treated suture.

5. Suture according to any one of the preceding claims, obtainable by thermal treatment of hydrolyzable suture with water vapor.

6. Suture according to Claim 5, **characterized in that** the thermal treatment is a pressurized steam treatment.

7. Suture according to any one of the preceding claims, **characterized in that** the initial knot-pull breaking strength is between 10 N and 90 N.

8. Suture based on polyglycolic acid according to any one of Claims 1 or 2 or 4 to 7, **characterized in that** it has a viscosity number of 0.45-0.65 dl/g.

9. Process for producing rapidly absorbable, surgical suture according to any one of Claims 1 to 8, **characterized in that** hydrolyzable suture based on polyglycolic acid or lactide copolymer is subjected to a thermal treatment with water vapor, the thermal treatment with water vapor is carried out in an autoclave and in the substantial absence of air, the autoclave is evacuated prior to the steam treatment, the thermal treatment is carried out at a treatment temperature between 120°C and 140°C, at a pressure between 2 and 5 bar and over a treatment duration of 4 min to 30 min and the suture is dried at between 60°C and 140°C after the thermal treatment and the drying is carried out under reduced pressure.

10. Process according to Claim 9, **characterized in that** the treatment is carried out with water vapor which is free of admixtures.

11. Process according to either of Claims 9 and 10, **characterized in that** the thermal treatment is carried out at a pressure between 2 bar and 3 bar.

12. Process according to any one of Claims 9 to 11, **characterized in that** the thermal treatment is carried out over a treatment duration of 4 min to 22 min.

13. Process according to Claim 9, **characterized in that** the drying is carried out at between 0.05 bar and 0.2 bar.

14. Process according to any one of Claims 9 to 13, **characterized in that** the thermal treatment is carried out with water vapor in a purely water vapor atmosphere.

15. Process according to any one of Claims 9 to 14, **characterized in that** ready-to-use suture is treated, in particular suture provided with a needle.

16. Suture obtainable by a process according to any one of Claims 9 to 15.

## Revendications

1. Matériau de suture chirurgical résorbable, traité thermiquement à la vapeur, à base d'acide polyglycolique ou d'un copolymère de lactide présentant une décomposition rapide et une diminution rapide de la force de rupture d'un noeud en conditions « in vivo » et une force de rupture d'un noeud initiale élevée en référence à la décomposition rapide, **caractérisé en ce que** la force de rupture d'un noeud initiale se situe dans la plage comprise entre 7 N et 95 N et la force de rupture d'un noeud du matériau de suture à base d'acide polyglycolique diminue en 14 jours à moins de 10 % de la force de rupture d'un noeud initiale et la force de rupture d'un noeud du matériau de suture à base d'un copolymère de lactide diminue en 14 jours à de 75 % à 90 % de la force de rupture d'un noeud initiale.

2. Matériau de suture à base d'acide polyglycolique selon la revendication 1, **caractérisé en ce que** la force de rupture d'un noeud diminue en 5 jours à moins de 65 % de la force de rupture d'un noeud initiale.

3. Matériau de suture à base d'un copolymère de lactide selon la revendication 1, **caractérisé en ce que** la force de rupture d'un noeud diminue en 28 jours à de 60 % à 75 % de la force de rupture d'un noeud initiale.

4. Matériau de suture selon l'une quelconque des revendications précédentes, **caractérisé en ce que** la force de rupture d'un noeud initiale est au moins 65 % de la force de rupture d'un noeud du matériau de suture non traité à la vapeur.

5. Matériau de suture selon l'une quelconque des revendications précédentes, pouvant être obtenu par traitement thermique d'un matériau de suture hydrolysable avec de la vapeur d'eau.

6. Matériau de suture selon la revendication 5, **caractérisé en ce que** le traitement thermique est un traitement à la vapeur sous pression.

7. Matériau de suture selon l'une quelconque des revendications précédentes, **caractérisé en ce que** la force de rupture d'un noeud initiale est comprise entre 10 N et 90 N.

8. Matériau de suture à base d'acide polyglycolique selon l'une quelconque des revendications 1 ou 2 ou 4 à 7, **caractérisé en ce qu'**il présente un indice de viscosité de 0,45 à 0,65 dl/g.

9. Procédé de fabrication d'un matériau de suture chirurgical rapidement résorbable selon l'une quelconque des revendications 1 à 8, **caractérisé en ce que** le matériau de suture hydrolysable à base d'acide polyglycolique ou d'un copolymère de lactide est soumis à un traitement thermique avec de la vapeur d'eau, le traitement thermique avec de la vapeur d'eau étant réalisé dans un autoclave et essentiellement sans air, l'autoclave étant évacué avant le traitement à la vapeur, le traitement thermique étant réalisé à une température de traitement comprise entre 120 °C et 140 °C, à une pression comprise entre 2 et 5 bar, pendant une durée de traitement de 4 min à 30 min, et le matériau de suture étant séché à une température comprise entre 60 °C et 140 °C après le traitement thermique, et le séchage étant réalisé sous pression réduite.

10. Procédé selon la revendication 9, **caractérisé en ce que** le traitement est réalisé avec de la vapeur d'eau qui est exempte d'additifs.

11. Procédé selon l'une quelconque des revendications 9 à 10, **caractérisé en ce que** le traitement thermique est réalisé à une pression comprise entre 2 bar et 3 bar.

12. Procédé selon l'une quelconque des revendications 9 à 11, **caractérisé en ce que** le traitement thermique est réalisé pendant une durée de traitement de 4 min à 22 min.

13. Procédé selon la revendication 9, **caractérisé en ce que** le séchage est réalisé entre 0,05 bar et 0,2 bar.

14. Procédé selon l'une quelconque des revendications 9 à 13, **caractérisé en ce que** le traitement thermique est réalisé avec de la vapeur d'eau dans une atmosphère de vapeur d'eau pure.

15. Procédé selon l'une quelconque des revendications 9 à 14, **caractérisé en ce qu'**un matériau de suture prêt à l'emploi est traité, notamment un matériau de suture déjà muni d'une aiguille.

16. Matériau de suture, pouvant être fabriqué par un procédé selon l'une quelconque des revendications 9 à 15.
